(19) Europäisches Patentamt
European Patent Office
Office européen des brevets

(11) EP 4 110 331 B1

(12) **EUROPEAN PATENT SPECIFICATION**

(45) Date of publication and mention of the grant of the patent:
**06.11.2024 Bulletin 2024/45**

(21) Application number: **21759790.5**

(22) Date of filing: **25.02.2021**

(51) International Patent Classification (IPC):
***A61K 31/4162*** *(2006.01)* ***A61K 31/519*** *(2006.01)*
***A61K 39/395*** *(2006.01)* ***A61P 35/00*** *(2006.01)*
***A61K 45/06*** *(2006.01)*

(52) Cooperative Patent Classification (CPC):
(C-Sets available)
**C07K 16/2887; A61K 31/519; A61K 45/06; A61P 35/00** (Cont.)

(86) International application number:
**PCT/CN2021/077935**

(87) International publication number:
**WO 2021/170045 (02.09.2021 Gazette 2021/35)**

(54) **METHODS OF TREATING DLBCL USING BTK INHIBITORS AND COMBINATIONS THEREOF**

VERFAHREN ZUR BEHANDLUNG VON DLBCL UNTER VERWENDUNG VON BTK-INHIBITOREN UND KOMBINATIONEN DAVON

MÉTHODES DE TRAITEMENT DE DLBCL À L'AIDE D'INHIBITEURS DE BTK ET DE LEURS COMBINAISONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2020 PCT/CN2020/077027**

(43) Date of publication of application:
**04.01.2023 Bulletin 2023/01**

(73) Proprietor: **Beigene Switzerland GmbH**
**4051 Basel (CH)**

(72) Inventors:
- **LIU, Yang**
  **Beijing 102206 (CN)**
- **WANG, Weige**
  **Beijing 102206 (CN)**
- **YAO, Hui**
  **Beijing 102206 (CN)**
- **HOU, Xinfeng**
  **Beijing 102206 (CN)**
- **GUO, Haiyi**
  **Beijing 102206 (CN)**
- **SHEN, Zhirong**
  **Beijing 102206 (CN)**
- **WANG, Lai**
  **Beijing 102206 (CN)**
- **YU, Yiling**
  **Beijing 102206 (CN)**

(74) Representative: **Mewburn Ellis LLP**
**Aurora Building**
**Counterslip**
**Bristol BS1 6BX (GB)**

(56) References cited:
**WO-A1-2015/127261 WO-A1-2018/033135**
**WO-A1-2018/033135 WO-A1-2019/108795**
**WO-A2-2018/033853 WO-A2-2018/033853**
**CN-A- 104 884 458**

- **WU JINGJING ET AL: "Second-generation inhibitors of Bruton tyrosine kinase", JOURNAL OF HEMATOLOGY & ONCOLOGY, vol. 9, no. 1, 2 September 2016 (2016-09-02), XP093024482, Retrieved from the Internet <URL:https://www.ncbi.nlm.nih.gov/pmc/articles/PMC5010774/pdf/13045_2016_Article_313.pdf> DOI: 10.1186/s13045-016-0313-y**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- "Report from the Virtual Edition of the 25th European Hematology Association (EHA), Annual Congress 11th -21st June 2020", MEMO - MAGAZINE OF EUROPEAN MEDICAL ONCOLOGY, SPRINGER VIENNA, VIENNA, vol. 13, no. Suppl 4, 1 October 2020 (2020-10-01), pages 91 - 110, XP037274194, ISSN: 1865-5041, [retrieved on 20201009], DOI: 10.1007/S12254-020-00652-7
- TARANTELLI CHIARA ET AL: "The Bruton tyrosine kinase inhibitor zanubrutinib (BGB-3111) demonstrated synergies with other anti-lymphoma targeted agents", HAEMATOLOGICA, vol. 104, no. 7, 24 January 2019 (2019-01-24), pages 307 - 309, XP055840282, Retrieved from the Internet <URL:https://www.ncbi.nlm.nih.gov/pmc/articles/PMC6601092/pdf/104e307.pdf> DOI: 10.3324/haematol.2018.214759
- CHEN RONGRONG ET AL: "MYD88 L265P and CD79B double mutations type (MCD type) of diffuse large B-cell lymphoma: mechanism, clinical characteristics, and targeted therapy", THERAPEUTIC ADVANCES IN HEMATOLOGY, vol. 13, 1 January 2022 (2022-01-01), GB, XP093076748, ISSN: 2040-6207, Retrieved from the Internet <URL:https://www.ncbi.nlm.nih.gov/pmc/articles/PMC8808040/pdf/10.1177_20406207211072839.pdf> DOI: 10.1177/20406207211072839
- TARANTELLI CHIARA, LU ZHANG, ELISABETTA CURTI, EUGENIO GAUDIO, FILIPPO SPRIANO, VALDEMAR PRIEBE ,LUCIANO CASCIONE, ALBERTO J ARRIB: "The Bruton tyrosine kinase inhibitor zanubrutinib (BGB-3111) demonstrated synergies with other anti-lymphoma targeted agents", HAEMATOLOGICA, vol. 104, no. 7, 24 January 2019 (2019-01-24), pages 307 - 309, XP055840282, DOI: 10.3324/haematol.2018.214759
- TAM CONSTANTINE S., TROTMAN JUDITH, OPAT STEPHEN, BURGER JAN A., CULL GAVIN, GOTTLIEB DAVID, HARRUP ROSEMARY, JOHNSTON PATRICK B.,: "Phase 1 study of the selective BTK inhibitor zanubrutinib in B-cell malignancies and safety and efficacy evaluation in CLL", BLOOD, AMERICAN SOCIETY OF HEMATOLOGY, US, vol. 134, no. 11, 12 September 2019 (2019-09-12), US, pages 851 - 859, XP055840284, ISSN: 0006-4971, DOI: 10.1182/blood.2019001160


(52) Cooperative Patent Classification (CPC): (Cont.)

C-Sets
**A61K 31/519, A61K 2300/00;**
**A61K 39/3955, A61K 2300/00**

## Description

### FIELD OF THE DISCLOSURE

Disclosed herein are methods of treating non-GCB DLBCL in a subject, comprising administering to the subject in need thereof (S)-7-(1-acryloylpiperidin-4-yl)-2-(4-phenoxyphenyl)-4,5,6,7-tetrahydropyrazolo-[1,5-a]pyrimidine-3-carboxamide or a pharmaceutically acceptable salt thereof. Disclosed herein are also methods of treating non-GCB DLBCL in a subject, comprising administering to the subject in need thereof (S)-7-(1-acryloylpiperidin-4-yl)-2-(4-phenoxyphenyl)-4,5,6,7-tetrahydropyrazolo-[1,5-a]pyrimidine-3-carboxamide or a pharmaceutically acceptable salt thereof, in combination with an anti-CD20 antibody.

### BACKGROUND OF THE DISCLOSURE

Diffuse large B-cell lymphoma (DLBCL), the most common subtype of non-Hodgkin's lymphoma, is clinically heterogeneous: 40% of patients respond well to current therapy and have prolonged survival, whereas the remainder succumb to the disease. (Alizadeh et al., Nature 2000;403(6769):503-11). DLBCL can be divided into prognostically significant subgroups with germinal center B-cell-like (GCB), activated B-cell-like (ABC), expression profiles (Alizadeh *supra*). The GCB group has a better survival rate than the ABC group, but both indications are associated with poor clinical outcomes. The use of molecular profiling to predict survival for DLBCL has seen previous use (Rosenwald et al., N Engl J Med. 2002;346: 1937-1947). In 2016, the World Health Organization (WHO) revised their classification of lymphoid neoplasms to account for the major advances in lymphoma biology since 2008 (Swerdlow et al., Blood. 2016;127(20):2375-2390). The WHO classification emphasized gene expression and alterations of clinical importance, such as changes in the MYC, BCL2, and/or BCL6 oncogenes. More than 80% of patients with the so-called "double-hit" lymphoma have translocations in MYC and BCL2 (Aukema et al., Blood. 2011;117(8):2319-2331). This indicates a need for additional molecular markers other than MYC and BCL2 that could be used to classify patients prior to treatment or to indicate response to treatment.

Bruton's tyrosine kinase (BTK) inhibitors alone or in combination with CD20 antibody only showed modest activity in R/R non-GCB DLBCL. However, patients with CD79B mutations responded to ibrutinib frequently (5/9; 55%), especially those with concomitant myeloid differentiation primary response 88 (MYD88) mutations (4/5; 80%). A phase 3 PHONIX trial of ibrutinib plus R-CHOP failed to meet its primary endpoints for treating frontline non-GCB DLBCL patients, however, biomarker analysis showed improved event-free survival in BCL2/MYC co-expression population. And these benefits were only observed in patients younger than 65 which also suggested a relatively high toxicity may be caused by the combinational use of ibrutinib and R-CHOP. So, these clinical outcomes indicate that patient stratification and patient response based on molecular markers will be the key for future non-GCB DLBCL treatment and regimens with less toxicity should be further investigated.

WO2015/127261 relates to methods for selecting patients diagnosed with diffuse large B-cell lymphoma (DLBCL) for treatment with an inhibitor of Bruton's tyrosine kinase (BTK) based on the level of expression the biomarkers CCL3 and/or CCL4.

### SUMMARY OF THE DISCLOSURE

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention.

WO2014/173289A disclosed a series of BTK inhibitors, particularly (S)-7-(1-acryloylpiperidin-4-yl)-2-(4-phenoxyphenyl)-4,5,6,7-tetra-hydropyrazolo[1,5-a]pyrimidine-3-carboxamide (hereinafter **Compound 1**). **Compound 1** can be used for the treatment of cancers with aberrations in the B-cell receptor (BCR) and FcR signaling pathway in which BTK plays important roles and has demonstrated to have potent and irreversible inhibitory activities against BTK.

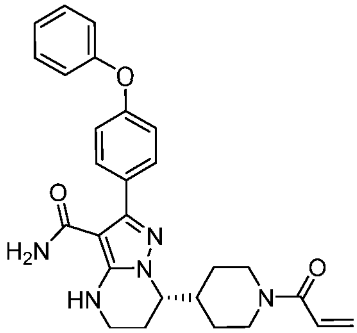

**Compound 1**

The present disclosure describes (S)-7-(1-acryloylpiperidin-4-yl)-2-(4-phenoxyphenyl)-4,5,6,7-tetra-hydropyrazolo[1,5-a]pyrimidine-3-carboxamide (**Compound 1**) or a pharmaceutically acceptable salt thereof alone, and a combination of **Compound 1** with anti-CD20 antibody, showed sensitive response in some populations of non-GCB DLBCL or ABC DLBCL, particularly those with a specific gene high expression, including PAX5, PIM1, BCL2, FOXP1, BCL2, MYC, or BCL2/MYC.

The present disclosure also describes **Compound 1** or a pharmaceutically acceptable salt thereof when administered alone, or administered as a combination of **Compound 1** with anti-CD20 antibody, showed sensitive response in some populations of non-GCB DLBCL, particularly those with a specific gene mutations, including mutations of BCR or NOTCH1 pathways, such as CD79B mutations or NOTCH1 mutations.

The inventors of the present disclosure discovered that ABC or non-GCB DLBCL is more sensitive to Compound 1 than GCB subtype in in-vitro and in-vivo assays.

In clinical studies, the inventors discovered that *PAX5* expression was significantly higher in **Compound 1** monotherapy responders, and *PIM1*, *BCL2*, and *FOXP1* expression was higher in **Compound 1** combination therapy responders. In **Compound 1** monotherapy, the inventors also discovered patients with *NOTCH1* mutations had higher ORR. In both **Compound 1** monotherapy and combination therapy of **Compound 1** in combined with anti-CD20 antibody, the inventors also discovered that patients with MYC, BCL2, MYC and BCL2 double expressor DLBCL tended to have higher ORR and longer progression-free survival and overall survival, and patients with *CD79B* showed significantly higher ORR than patients without *CD79B* mutations.

In addition, the inventors of present disclosure discovered that EBV negative non-GCB DLBCL were more sensitive to **Compound 1** than EBV positive non-GCB DLBCL.

In a first aspect, the present invention relates to a BTK inhibitor for use in a method of treating a human patient with non-GCB diffuse large B-cell lymphoma, wherein the patient is characterized by a CD79B mutation, and wherein the BTK inhibitor is Compound 1.

In a second aspect, the present invention relates to a BTK inhibitor for use in a method of treating a human patient with non-GCB diffuse large B-cell lymphoma, wherein the BTK inhibitor is administered in combination with an anti-CD20 antibody, wherein the patient is characterized by a CD79B mutation, and wherein the BTK inhibitor is Compound 1.

Articles of manufacture, or "**kits**" comprising a first container, a second container and a package insert, wherein the first container comprises at least one dose of a medicament comprising **Compound 1** or a pharmaceutically acceptable salt thereof, the second container comprises at least one dose of a medicament comprising an anti-CD20 antibody, and the package insert comprises instructions for treating non-GCB DLBCL in a subject using the medicaments is also included.

In one embodiment, the anti-CD20 antibody is selected from rituximab, ibritumomab tiuxetan, tositumomab, ofatumumab or obinutuzumab. The non-GCB DLBCL is a non-GCB DLBCL with a CD79B mutation

In one embodiment of each of the above aspects, the non-GCB is a R/R non-GCB DLBCL.

In one embodiment of each of the above aspects, the non-GCB is a R/R ABC DLBCL.

## BRIEF DESCRIPTION OF THE DRAWINGS

**Figure 1** showed ABC/non-GCB DLBCL is more sensitive to **Compound 1** than GCB subtype.

DLBCL PDX models were classified into GCB and non-GCB subtypes and treated with compound 1 as described in methods. Tumor growth inhibition (TGI) of compound 1 in each mice was calculated. Each dot points represent

the mean TGI of one model. P<0.05 (two tails t test).

**Figure 2** showed non-GCB DLBCL PDX models with CD79B mutation, or CD79B-ITAM domain (amino acid 193-210) mutation are more sensitive to Compound 1.

**Figure 2(A):** non-GCB DLBCL PDX models were treated with **Compound 1** and TGI of each model were calculated described in methods. The models were subdivided into CD79B wildtype (CD79B-WT) and CD79B mutation (CD79B-mut) groups. Each dot points represent the mean TGI of one model. P<0.05 (two tails t test).

Figure 2(B): non-GCB DLBCL PDX models were treated with **Compound 1** and TGI of each model were calculated described in methods. The models were subdivided into CD79B ITAM domain (amino acid 193-210) wildtype (CD79B ITAM-WT) and CD79B ITAM domain mutation (CD79B ITAM-mut) groups. Each dot points represent the mean TGI of one model. P<0.05 (two tails t test).

**Figure 3** showed EBV negative non-GCB DLBCL PDX models are more sensitive to **Compound 1.**

Non-GCB DLBCL PDX models were treated with **Compound 1** and TGI of each mice were calculated as described in methods. The models were subdivided into Epstein-Barr virus (EBV) negative (EBV-) and EBV positive (EBV+) groups. Each dot points represent the mean TGI of one model. P<0.05 (two tails t test).

**Figure 4** showed 56 non-GCB DLBCL subtyped by the GEP method were subjected to mRNA analysis (total 95 genes).

**Figure 4(A)** showed PAX5 was enriched in responders in **Compound 1** monotherapy schedules 1 and 2.

**Figure 4(B)** showed PIM1, FOXP1 and BCL2 were enriched in responders to **Compound 1** in combination with CD20 antibody via schedules 3 and 4.

**Figure 5** showed best observed response (BOR), progression free survival (PFS) and overall survival (OS) in 28 BCL2-high and 28 BCL2-low patients(median expression value of BCL2 was used as the threshold for defining BCL2-high or BCL2-low).

**Figure 6** showed best observed response (BOR), progression free survival (PFS) and overall survival (OS) in 28 MYC-high and28 MYC-low patients(median expression value of MYC was used as the threshold for defining MYC-high or MYC-low).

**Figure 7** showed best observed response (BOR), progression free survival (PFS) and overall survival (OS) in 18 DE patients and 38 non-DE patients (Median expression value of BCL2/MYC were used as threshold for defining DE or non-DE).

## DETAILED DESCRIPTION OF THE DISCLOSURE

### Definitions

Unless specifically defined elsewhere in this document, all other technical and scientific terms used herein have the meaning commonly understood by one of ordinary skill in the art.

As used herein, including the appended claims, the singular forms of words such as "**a**," "**an,**" and "**the,**" include their corresponding plural references unless the context clearly dictates otherwise.

The term "**or**" is used to mean, and is used interchangeably with, the term "**and/or**" unless the context clearly dictates otherwise.

The terms "**administration,**" "**administering,**" "**treating,**" and "**treatment**" herein, when applied to an animal, human, experimental subject, cell, tissue, organ, or biological fluid, means contact of an exogenous pharmaceutical, therapeutic, diagnostic agent, or composition to the animal, human, subject, cell, tissue, organ, or biological fluid. Treatment of a cell encompasses contact of a reagent to the cell, as well as contact of a reagent to a fluid, where the fluid is in contact with the cell. The term "administration" and "treatment" also means *in vitro* and *ex vivo* treatments, e.g., of a cell, by a reagent, diagnostic, binding compound, or by another cell. The term "subject" herein includes any organism, preferably an animal, more preferably a mammal (e.g., rat, mouse, dog, cat, rabbit) and most preferably a human. Treating any disease or disorder refer in one aspect, to ameliorating the disease or disorder (i.e., slowing or arresting or reducing the development of the disease or at least one of the clinical symptoms thereof). In another aspect, "treat," "treating," or "treatment" refers to alleviating or ameliorating at least one physical parameter including those which may not be discernible by the patient. In yet another aspect, "treat," "treating," or "treatment" refers to modulating the disease or disorder, either physically, (e.g., stabilization of a discernible symptom), physiologically, (e.g., stabilization of a physical parameter), or both. In yet another aspect, "treat," "treating," or "treatment" refers to preventing or delaying the onset or development or progression of the disease or disorder.

The term "**therapeutically effective amount**" as herein used, refers to the amount of a Bcl-2 inhibitor that, when administered to a subject for treating a disease, or at least one of the clinical symptoms of a disease or disorder, is sufficient to effect such treatment for the disease, disorder, or symptom. The "therapeutically effective amount" can vary with the agent, the disease, disorder, and/or symptoms of the disease or disorder, severity of the disease, disorder, and/or symptoms of the disease or disorder, the age of the subject to be treated, and/or the weight of the subject to be

treated. An appropriate amount in any given instance can be apparent to those skilled in the art or can be determined by routine experiments. In the case of combination therapy, the "therapeutically effective amount" refers to the total amount of the combination objects for the effective treatment of a disease, a disorder or a condition.

The present invention provides a BTK inhibitor for use in a method of treating a human patient with non-GCB diffuse large B-cell lymphoma, wherein the patient is characterized by a CD79B mutation, and wherein the BTK inhibitor is Compound 1.

The present invention also provides a BTK inhibitor for use in a method of treating a human patient with non-GCB diffuse large B-cell lymphoma, wherein the BTK inhibitor is administered in combination with an anti-CD20 antibody, wherein the patient is characterized by a CD79B mutation, and wherein the BTK inhibitor is Compound 1.

## Anti-CD20 antibody

The "anti-CD20 antibody" includes, but not limited to, rituximab, ibritumomab tiuxetan, tositumomab, ofatumumab or obinutuzumab.

## Methods of Treatment

In one aspect, the present disclosure provides a method of treating non-GCB DLBCL or ABC DLBCL in a human subject, wherein said subject is characterized by a CD79B mutation.

The method comprises administering to the human subject in need thereof **Compound 1** or a pharmaceutically acceptable salt thereof.

The non-GCB DLBCL is a non-GCB DLBCL with a CD79B mutation

In yet another aspect, disclosed herein is **Compound 1** or a pharmaceutically acceptable salt thereof, for use in the treatment of non-GCB DLBCL or ABC DLBCL, in combination with an anti-CD20 antibody. The non-GCB DLBCL is a non-GCB DLBCL with a CD79B mutation

**Compound 1** can be administered by any suitable means, including oral, parenteral, intrapulmonary, and intranasal, and, if desired for local treatment, intralesional administration. Dosing can be by any suitable route. Various dosing schedules including but not limited to single or multiple administrations over various time-points, bolus administration, and pulse infusion are contemplated herein.

**Compound 1** would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

For the prevention or treatment of disease, the appropriate dosage of **Compound 1** and anti-CD20 antibody in a monotherapy or in a combination therapy will depend on the type of disease to be treated, the severity and course of the disease.

In some embodiments, **Compound 1** is orally administrated at dose of 40 mg-640mg per day. In some embodiments, **Compound 1** is orally administrated at dose of 100 mg-400mg per day. In some embodiments, **Compound 1** is orally administrated at dose of 320 mg QD or 160 mg BID.

In some embodiments, the anti-CD20 antibody is obinutuzumab. In some embodiments, Compound 1 is orally administrated at a dose of 320 mg QD or 160 mg BID in 28-day cycles, and obinutuzumab is administrated by three loading doses of 1000 mg weekly followed by 1000 mg on day one of cycles 2-6.

In some embodiments, the anti-CD20 is rituximab. **Compound 1** was administrated in combination with rituximab. In some embodiments, **Compound 1** is orally administrated at a dose of 320 mg QD or 160 mg BID, rituximab is administered at 375 mg/m2 intravenously on Cycle 1 Days 1, 8, 15, 22, and on Day 1 of Cycles 4, 6, 8, 10, and **Compound 1** is administered at least 30 minutes prior to the initiation of the rituximab infusion.

## ABBREVIATIONS AND DEFINITIONS OF TERMS

| Abbreviation | Term |
|---|---|
| ABC-DLBCL | activated B cell-like DLBCL |
| BID | twice daily |
| BOR | best overall response |

(continued)

| Abbreviation | Term |
|---|---|
| CLL | chronic lymphocytic leukemia |
| CR | complete response |
| DE | Double Expressor |
| DLBCL | diffuse large B-cell lymphoma |
| DOR | duration of response |
| GCB | germinal center B-cell-like |
| NGS | next generation sequencing |
| ORR | overall response rate |
| **OS** | overall survival |
| **PD** | progressive disease |
| **PFS** | progression-free survival |
| **PR** | partial response |
| **PT** | preferred term |
| **QD** | once daily |
| **R/R** | relapsed/refractory |

## EXAMPLES

The present invention is further exemplified, but not limited to, by the following examples that illustrate the invention.

### Example 1 Comparison of DLBCL cell of origin (COO) to the influence of Compound 1 response

***Methods***

DLBCL patient-derived xenografted (PDX) models were established in immunodeficient NCG (NOD-Prkdcem26Cd52Il2rgem26Cd22/NjuCrl) mice. These models were classified into GCB and non-GCB subgroups based on immunohistochemistry (IHC) staining using Hans algorithm (Hans, C.P., et al., Blood, 2004. 103 (1): p.275-282.). For efficacy studies, tumor tissues were inoculated subcutaneously into NCG mice. When tumors reached 100~200 $mm^3$, mice were randomized into each group with 7~10 mice/group based on tumor volume. **Compound 1** was formulated with 0.5% methyl cellulose as vehicle and treatment was started on the day of randomized grouping. Compound 1 was administrated p.o., 7.5mg/(kg body weight), BID. Tumor volumes were measured twice weekly in two dimensions using a caliper, and the volume were expressed in $mm^3$ using the formula:
$V = 0.5(a \times b^2)$ where *a* and *b* are the long and short diameters of the tumor, respectively. Tumor growth inhibition (TGI) was calculated using the following formula:

$$\%\ TGI = 100 \times [1 - (treatment_{vt} - treatment_{v0}) / (vehicle_{vt} - Vehicle_{v0})]$$

$Treatment_{vt}$ = tumor volume of treatment group at time *t*.
$treatment_{v0}$ = tumor volume of treatment group at time of treatment started.
$Vehicle_{vt}$ = tumor volume of vehicle group at time t.
$Vehicle_{v0}$ = tumor volume of vehicle group at time of treatment started.
P value was calculated using two tails t test.

***Results***

**Compound 1** resulted in higher TGI in non-GCB DLBCL (TGI 63.60 ± 42.92, mean±SD) models than that of GCB subtype (TGI 25.5±11.96, mean±SD), $P<0.05$ (two tails t test). See **Figure 1.**

***Conclusion***

DLBCL of non-GCB subtype were significantly more sensitive to Compound 1 than those of GCB subtype.

**Example 2 Comparison of CD79B and CD79B ITAM domain mutation to the sensitivity of compound 1 in DLBCL**

***Methods***

DLBCL patient-derived xenografted (PDX) models were established in immunodeficient NCG (NOD-Prkdcem26Cd52Il2rgem26Cd22/NjuCrl) mice. These models were classified into GCB and non-GCB subgroups based on immunohistochemistry (IHC) staining using Hans algorithm. Non-GCB DLBCL PDX models were classified into CD79B mutant versus CD79B wildtype groups; or CD79B ITAM domain mutant (amino acid 193-210) versus CD79B ITAM domain wildtype groups. For efficacy studies, tumor tissues were inoculated subcutaneously into NCG mice. When tumors reached 100~200 $mm^3$, mice were randomized into each group with 7~10 mice/group based on tumor volume. Compound 1 was formulated with 0.5% methyl cellulose as vehicle and treatment was started on the day of randomized grouping. Compound 1 was administrated p.o., 7.5mg/(kg body weight), BID. Tumor volumes were measured twice weekly in two dimensions using a caliper, and the volume were expressed in $mm^3$ using the formula:
$V = 0.5(a \times b^2)$ where *a* and *b* are the long and short diameters of the tumor, respectively. Tumor growth inhibition (TGI) was calculated using the following formula:

$$\% \text{ TGI} = 100 \times [1 - (\text{treatment}_{vt} - \text{treatment}_{v0}) / (\text{vehicle}_{vt} - \text{Vehicle}_{v0})]$$

$\text{Treatment}_{vt}$ = tumor volume of treatment group at time *t*.
$\text{treatment}_{v0}$ = tumor volume of treatment group at time of treatment started.
$\text{Vehicle}_{vt}$ = tumor volume of vehicle group at time *t*.
$\text{Vehicle}_{v0}$ = tumor volume of vehicle group at time of treatment started.
P value was calculated using two tails t test.

***Results***

**Compound 1** resulted in higher TGI in CD79B mutation (TGI 87.5±33.14, mean±SD) or CD79B ITAM domain mutation (TGI 96.00±28.83, mean±SD) non-GCB DLBCL models than that of CD79B wildtype (TGI 49.44±42.36, mean±SD) or CD79B ITAM domain wildtype (TGI 49.18±41.03, mean±SD) non-GCB DLBCL models, $P<0.05$ (two tails t test). See **Figure 2.**

***Conclusion***

Non-GCB DLBCL with CD79B gene mutation were more sensitive to **Compound 1** than those with wildtype CD79B gene. Non-GCB DLBCL with CD79B ITAM domain mutation were more sensitive to compound 1 than those without CD79B ITAM domain mutation.

**Example 3 Comparison of EBV infection status to the sensitivity of compound 1 in**

**DLBCL**

***Methods***

DLBCL patient-derived xenografted (PDX) models were established in immunodeficient NCG (NOD-Prkdcem26Cd52Il2rgem26Cd22/NjuCrl) mice. These models were classified into GCB and non-GCB subgroups based on immunohistochemistry (IHC) staining using Hans algorithm. Non-GCB DLBCL PDX models were classified into Epstein-Barr virus (EBV) negative and EBV positive subgroups. For efficacy studies, tumor tissues were inoculated subcutaneously into NCG mice. When tumors reached 100~200 $mm^3$, mice were randomized into each group with 7~10

mice/group based on tumor volume. Compound 1 was formulated with 0.5% methyl cellulose as vehicle and treatment was started on the day of randomized grouping. Compound 1 was administrated p.o., 7.5mg/(kg body weight), BID. Tumor volumes were measured twice weekly in two dimensions using a caliper, and the volume were expressed in $mm^3$ using the formula:
$V = 0.5(a \times b^2)$ where $a$ and $b$ are the long and short diameters of the tumor, respectively. Tumor growth inhibition (TGI) was calculated using the following formula:

$$\% \text{ TGI} = 100 \times [1 - (\text{treatment}_{vt} - \text{treatment}_{v0}) / (\text{vehicle}_{vt} - \text{Vehicle}_{v0})]$$

$\text{Treatment}_{vt}$ = tumor volume of treatment group at time *t*.
$\text{treatment}_{v0}$ = tumor volume of treatment group at time of treatment started.
$\text{Vehicle}_{vt}$ = tumor volume of vehicle group at time *t*.
$\text{Vehicle}_{v0}$ = tumor volume of vehicle group at time of treatment started.

Average TGI of each model was used and represented as a dot point in the figure. P value was calculated using two tails t test to compare difference between groups.

***Results***

**Compound 1** resulted higher TGI in EBV negative (TGI 78.00±43.41, mean±SD) non-GCB DLBCL models than that of EBV positive (TGI 33.25±22.41, mean±SD) models, P<0.05 (two tails t test). See **Figure 3.**

***Conclusion***

EBV negative non-GCB DLBCL were more sensitive to **Compound 1** than EBV positive non-GCB DLBCL.

**Example 4 Treatment of non- germinal center B-cell like diffuse large B-cell lymphomas (non-GCB DLBCL) with BTK inhibitor Treatment**

A total of 121 patients with R/R non-GCB DLBCL were recruited in four **Compound 1** treatment studies that were conducted at a similar time period. Two of the four studies were **Compound 1** monotherapy (n = 79) and two were **Compound 1** combined with an anti-CD20 antibody therapy (n = 42). Similar inclusion and exclusion criteria and response evaluation criteria were used across all studies. Fifty-six non-GCB patients were further subtyped by gene expression profiling (GEP) using the HTG EdgeSeq DLBCL Cell of Origin Assay. The expression of approximately 90 lymphoma-associated genes from the HTG GEP assay were analyzed by R package limma for correlation with response to **Compound 1** treatment. Seventy-seven patient samples were tested by next-generation sequencing (NGS) with a panel of genes. Chi-square test was used to evaluate the correlation between mutations and the objective response rate (ORR).

**Example 4A: Treatment of non-GCB DLBCL with BTK inhibitor monotherapy *Methods***

Monotherapy schedule 1: **Compound 1** was administered orally every day at 160 mg twice daily (BID) in 28-day cycles in patients with R/R non-GCB DLBCL .
Monotherapy schedule 2: **Compound 1** was administered orally every day in each cycle at 160 mg BID in 28-day cycles in patients with R/R non-GCB DLBCL.
79 patients with non-GCB DLBCL were subjected to **Compound 1** monotherapies (Monotherapy Schedule 1 or 2).

***Results***

The unadjusted ORR in non-GCB DLBCL was 31.6% and 29.3% for the two monotherapies. For patients who GEP-confirmed activated B-cell DLBCL classification, the ORR was 53.8% and 36%, respectively.
For the 44 non-GCB patients with HTG gene expression profiles, *PAX5* expression was significantly higher in the two monotherapies responders. (See **Figure 4**).
For the 44 patients with non-GCB DLBCL underwent panel sequencing, the mutations of B-cell receptor (BCR) pathway or NOTCH1 pathway related genes were identified to be correlated with better response. Patients with non-GCB DLBCL with CD79B mutations (n=17) showed higher ORR than other population (52.9% vs. 33.3%, p=0.2). Patients with NOTCH1 mutant (n=3) had higher ORR in the two monotherapies (100% vs. 36.6%, p<0.05). *See* **Table 1.**

**Table 1**

| non-GCB | Compound 1 Monotherapy | | |
|---|---|---|---|
| | 160 BID (Schedule 1; N=38) Response/Patient ORR | 160 BID (Schedule 2; N=41) Response/Patients ORR | Total (N=79) Response/ Patients ORR |
| Overall Response | | | |
| Non-GCB DLBCL Patients | 12/38 31.6 | 12/41 29.3 | 24/79 30.4 |
| Patients with Sequence Available | 7/11 63.6 | 12/34 35.3 | 19/45 42.2 |
| Non-GCB DLBCL Patients with Sequence Available | 6/10 60.0 | 12/34 35.3 | 18/44 40.9 |
| ABC Subtype of DLBCL Patien ts | 7/13 53.8 | 9/25 36.0 | 16/38 42.1 |
| **NOTCH1** | | | |
| NOTCH1_Mut | 1/1 100.0 | 2/2 100.0 | 3/3 100.0 |
| NOTCH1_WT | 5/9 55.6 | 10/32 31.3 | 15/41 36.6 |
| Difference in ORR (95% CI) [1] | 44.4 (-45.95,74.47) | 68.8 (-0.40,82.20) | 63.4 (4.48,76.54) |
| Two-sided P-value | 0.3894 | 0.0484 | 0.0310 |
| **MYD88/ CD79B** | | | |
| MYD88_L265P/CD79B Mut | | 3/6 50.0 | 3/6 50.0 |
| Other | 6/10 60.0 | 9/28 32.1 | 15/38 39.5 |
| Difference in ORR (95% CI) [1] | | 17.9 (-20.09,54.50) | 10.5 (-25.93,46.49) |
| Two-sided P-value | | 0.4062 | 0.6260 |
| **MYD88 mutation** | | | |
| MYD88 L265P | 0/1 0.0 | 4/10 40.0 | 4/11 36.4 |
| MYD88_WT | 6/9 66.7 | 8/24 33.3 | 14/33 42.4 |
| Difference in ORR (95% CI) [1] | -66.7 (-88.57,24.79) | 6.7 (-25.72,41.25) | -6.1 (-34.77,27.31) |
| Two-sided P-value | 0.1967 | 0.7109 | 0.7233 |
| **CD79B mutation** | | | |
| CD79B Mut | 3/4 75.0 | 6/13 46.2 | 9/17 52.9 |
| CD79B WT | 3/6 50.0 | 6/21 28.6 | 9/27 33.3 |
| Difference in ORR (95% CI) [1] | 25.0 (-37.11,69.78) | 17.6 (-15.11,48.53) | 19.6 (-10.20,46.87) |
| Two-sided P-value | 0.4292 | 0.2972 | 0.1977 |
| ***CD79B Functional mutation*** | | | |
| CD79B ITAM Mut | 2/2 100.0 | 6/13 46.2 | 8/15 53.3 |
| CD79B ITAM WT | 4/8 50.0 | 6/21 28.6 | 10/29 34.5 |
| Difference in ORR (95% CI) [1] | 50.0 (-28.56,79.49) | 17.6 (-15.11,48.53) | 18.9 (-11.55,46.83) |

(continued)

| *CD79B Functional mutation* | | | |
|---|---|---|---|
| Two-sided P-value | 0.1967 | 0.2972 | 0.2280 |
| [1] difference 95% CI were based on Miettinen and Nuriminen method<br>[2] chi-square test | | | |

***Conclusion***

Patients with higher expression of PAX5 or mutations of NOTCH1 or CD79B showed better response upon administration of **Compound 1** in monotherapy treatment regimens. As such, screening of non-GCB DLBCL patients for *PAX5* expression or NOTCH1 or CD79B mutations before treatment is a useful marker in determining patient response to **Compound 1** and whether treatment with **Compound 1** should be continued.

**Example 4B: Treatment of non-GCB DLBCL with a BTK inhibitor in combination with an anti-CD20 antibody**

***Methods***

Combination therapy schedule 1: **Compound 1** was administered orally every day in each cycle at 160 mg BID in 28-day cycles, in combination with obinutuzumab, in patients with R/R non-GCB DLBCL. Obinutuzumab was administered for up to 6 cycles consistent with the U.S. label regimen (100 mg at Day 1 Cycle 1, 1000mg at Day 8 and Day 15, 900 mg at Day 2 Cycle1 followed by 1000 mg on day one of cycles 2-6).

Combination therapy schedule 2: **Compound 1** was administrated orally every day in each cycle at 160 mg BID in 28-day cycles, in combination with rituximab, in patients with R/R non-GCB DLBCL. Rituximab was administered at 375 mg/m2 intravenously on Cycle 1 Days 1, 8, 15, 22, and on Day 1 of Cycles 4, 6, 8, 10. **Compound 1** was administered at least 30 minutes prior to the initiation of the rituximab infusion.

42 patients with non-GCB DLBCL were subjected to **Compound 1** plus anti-CD20 antibody Combination therapy schedule 1 or 2.

***Results***

The unadjusted ORR in non-GCB DLBCL was 22.7% and 35% for the two combo-therapies. For patients who GEP-confirmed activated B-cell DLBCL classification, the ORR was 50% and 40%, respectively.

The expression of approximately 90 lymphoma-associated genes from the HTG GEP assay were analyzed by R package limma for correlation with response to Combination therapy schedules 1 to 2. For the 12 non-GCB patients with HTG gene expression profiles, *PIM1, BCL2,* and *FOXP1* expression was higher in combination therapy responders. (See **Figure 4**).

Patients with non-GCB DLBCL with *CD79B* mutations (n=8) showed significantly higher ORR than patients without *CD79B* mutations. (see **Table 2**).

**Table 2**

| **non-GCB** | **Compound 1 Combination therapy** | | |
|---|---|---|---|
| | **Plus obinutuzumab (N=22) Response/Patients ORR** | **Plus** rituximab **(N=20) Response/Patients ORR** | **Total (N=42) Response/Patients ORR** |
| Overall Response | | | |
| Non-GCB DLBCL Patients | 5/22 22.7 | 7/20 35.0 | 12/42 28.6 |
| Patients with Sequence Available | 5/16 31.3 | 7/18 38.9 | 12/34 35.3 |
| Non-GCB DLBCL Patients with Sequence Available | 4/15 26.7 | 7/18 38.9 | 11/33 33.3 |
| ABC Subtype of DLBCL Patient s | 3/6 50.0 | 2/5 40.0 | 5/11 45.5 |

(continued)

| | | | |
|---|---|---|---|
| **NOTCH1** | | | |
| NOTCH1_Mut | 2/3 66.7 | 0/2 0.0 | 2/5 40.0 |
| NOTCH1_WT | 2/12 16.7 | 7/16 43.8 | 9/28 32.1 |
| Difference in ORR (95% CI) [1] | 50.0 (-6.15,84.24) | -43.8 (-67.39,28.69) | 7.9 (-27.98,49.62) |
| Two-sided P-value | 0.0798 | 0.2315 | 0.7314 |
| **MYD88/ CD79B** | | | |
| MYD88 L265P/CD79B Mut | 3/3 100.0 | 0/1 0.0 | 3/4 75.0 |
| Other | 1/12 8.3 | 7/17 41.2 | 8/29 27.6 |
| Difference in ORR (95% CI) [1] | 91.7 (29.61,98.58) | -41.2 (-64.57,44.19) | 47.4 (-2.08,74.68) |
| Two-sided P-value | 0.0013 | 0.4117 | 0.0593 |
| **MYD88 mutation** | | | |
| MYD88_L265P | 3/3 100.0 | 1/3 33.3 | 4/6 66.7 |
| MYD88_WT | 1/12 8.3 | 6/15 40.0 | 7/27 25.9 |
| Difference in ORR (95% CI) [1] | 91.7 (29.61,98.58) | -6.7 (-47.89,47.64) | 40.7 (-1.36,70.35) |
| Two-sided P-value | 0.0013 | 0.8288 | 0.0555 |
| **CD79B mutation** | | | |
| CD79B_Mut | 3/3 100.0 | 3/5 60.0 | 6/8 75.0 |
| CD79B_WT | 1/12 8.3 | 4/13 30.8 | 5/25 20.0 |
| Difference in ORR (95% CI) [1] | 91.7 (29.61,98.58) | 29.2 (-19.61,67.42) | 55.0 (15.75,78.76) |
| Two-sided P-value | 0.0013 | 0.2545 | 0.0041 |
| ***CD79B Functional mutation*** | | | |
| CD79B ITAM Mut | 3/3 100.0 | 2/3 66.7 | 5/6 83.3 |
| CD79B ITAM_WT | 1/12 8.3 | 5/15 33.3 | 6/27 22.2 |
| Difference in ORR (95% CI) [1] | 91.7 (29.61,98.58) | 33.3 (-22.23,71.01) | 61.1 (17.03,82.01) |
| Two-sided P-value | 0.0013 | 0.2796 | 0.0041 |
| [1] difference 95% CI were based on Miettinen and Nuriminen method<br>[2] chi-square test | | | |

***Conclusion***

The combination therapy results were very different from the monotherapy results. The enrichment of PAX5 expression upon administration of the mono-therapy was not noted. Instead, increased expression of PIM1, BCL2, and FOXP1 was seen for the Combination Therapy patients. Patients with CD79B mutations showed significantly better response to combo-therapies. Therefore, screening of non-GCB DLBCL patients for PIM1, BCL2, or FOXP1 expression or CD79B mutation before treatment are useful markers in determining patient response to **Compound 1** with an anti-CD20 antibody and whether treatment with the combination should be continued.

**Example 4C: Treatment of non-GCB DLBCL with BTK inhibitor monotherapy, or combination therapy**

***Methods***

Monotherapy schedule 1: **Compound 1** was administered orally every day at 160 mg twice daily (BID) in 28-day cycles in patients with R/R non-GCB DLBCL

Monotherapy schedule 2: **Compound 1** was administered orally every day in each cycle at 160 mg BID in 28-day cycles in patients with R/R non-GCB DLBCL.

Combination therapy schedule 1: **Compound 1** was administered orally every day in each cycle at 160 mg BID in 28-day cycles, in combination with obinutuzumab, in patients with R/R non-GCB DLBCL. Obinutuzumab was administered for up to 6 cycles consistent with the U.S. label regimen (100 mg at Day 1 Cycle 1, 1000mg at Day 8 and Day 15, 900 mg at Day 2 Cycle1 followed by 1000 mg on day one of cycles 2-6).

Combination therapy schedule 2: **Compound 1** was administrated orally every day in each cycle at 160 mg BID in 28-day cycles, in combination with rituximab, in patients with R/R non-GCB DLBCL. And, rituximab was administered at 375 mg/m2 intravenously on Cycle 1 Days 1, 8, 15, 22, and on Day 1 of Cycles 4, 6, 8, 10. **Compound 1** was administered at least 30 minutes prior to the initiation of the rituximab infusion.

A total of 121 patients with non-GCB DLBCL were included for response evaluation. 79 patients were subjected to **Compound 1** monotherapies (Monotherapy Schedule 1 or 2), and 42 patients were subjected to **Compound 1** plus anti-CD20 antibody Combination therapy (Combination therapy schedule 1 or 2) .

56 non-GCB patients were further subtyped by gene expression profiling (GEP) using the HTG EdgeSeq DLBCL Cell of Origin Assay. The expression of approximately 90 lymphoma-associated genes from the HTG GEP assay were analyzed by R package limma for correlation with response to zanubrutinib treatment.

***Results***

Median expression value of BCL2 was used as threshold for defining BCL2-high and BCL2-low groups. Total 28 BCL2-high (BCL2-H) and 28 BCL2-low (BCL2-L) patients were investigated. Patients high BCL2 expression had significantly better BOR (16/28, 57% vs. 6/28, 21%, p=0.028) and longer PFS (5.3m vs. 2.8m, p=0.03)/OS (10m vs. 6m, p=0.21). See **Figures 5A, 5B** and **5C.**

Median expression value of MYC was used as threshold for defining MYC-high and MYC-low groups. Total 28 MYC-high and 28 MYC-low patients were investigated. Patients high MYC expression tended to have better BOR (13/28, 46% vs. 9/28, 32%, p=0.14) and longer PFS (5.4m vs. 2.9m, p=0.52)/OS (10m vs. 7m, p=0.48). See **Figures 6A, 6B** and **6C.**

Median expression value of BCL2/MYC were used as threshold for defining double expressor (DE) or non-DE. Patients (18 DE and 38 non-DE) were subjected to the two monotherapies and the two Combination therapies. Patients with MYC and BCL2 double expressor DLBCL tended to have higher ORR (11/18, 61% vs 11/38, 29%; P = 0.12) and longer progression-free survival (5.4 months vs 3.6 months; P = 0.16) and overall survival (10 months vs 7 months, P = 0.32). See **Figures 7A, 7B,** and **7C.**

***Conclusion***

Patients with *MYC*, *BCL2*, or *BCL2*/ *MYC* double expressor DLBCL tended to have higher ORR and longer progression-free and overall survival with higher response rates to in the four **Compound 1** treatment studies. Hence, screening of non-GCB DLBCL patients for *MYC*, *BCL2*, or *BCL2*/ *MYC* double expressor expression after treatment is a useful marker in determining patient response to **Compound 1** and **Compound 1** with an anti-CD20 antibody, and whether treatment with the monotherapy or combination therapy should be continued.

The foregoing examples and description of certain embodiments should be taken as illustrating, rather than as limiting the present invention as defined by the claims.

**Claims**

1. A BTK inhibitor for use in a method of treating a human patient with non-GCB diffuse large B-cell lymphoma, wherein the patient is **characterized by** a CD79B mutation, and wherein the BTK inhibitor is **Compound 1:**

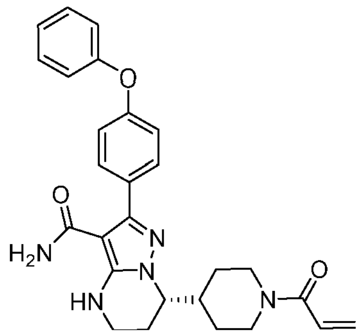

**Compound 1.**

2. The BTK inhibitor for use according to claim 1, wherein Compound 1 is administered at 320 mg once daily.

3. The BTK inhibitor for use according to claim 1, wherein Compound 1 is administered at 160 mg twice daily.

4. A BTK inhibitor for use in a method of treating a human patient with non-GCB diffuse large cell lymphoma, wherein the BTK inhibitor is administered in combination with an anti-CD20 antibody, wherein the patient is **characterized by** a CD79B mutation, and wherein the BTK inhibitor is **Compound 1:**

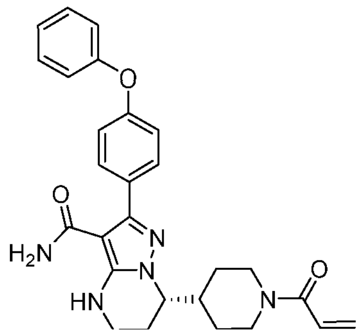

**Compound 1.**

5. The BTK inhibitor for use according to claim 4, wherein Compound 1 is administered at 320 mg once daily.

6. The BTK inhibitor for use according to claim 4, wherein Compound 1 is administered at 160 mg twice daily.

7. The BTK inhibitor for use according to claim 2, 3, 5 or 6, wherein Compound 1 is administered in 28 day cycles.

8. The BTK inhibitor for use according to claim 4, wherein the anti-CD20 antibody is rituximab, ibritumomab tiuxetan, tositumomab, ofatumumab or obinutuzumab.

9. The BTK inhibitor for use according to claim 8, wherein the anti-CD20 antibody is rituximab.

10. The BTK inhibitor for use according to claim 8, wherein the anti-CD20 antibody is obinutuzumab.

11. An anti-CD20 antibody for use in a method of treating a human patient with non-GCB diffuse large B-cell lymphoma, wherein a BTK inhibitor is administered in combination with the anti-CD20 antibody, wherein the patient is **characterized by** a CD79B mutation, wherein the BTK inhibitor is Compound 1 of claim 4,

optionally wherein Compound 1 is administered at 320 mg once daily or at 160 mg twice daily,
optionally wherein Compound 1 is administered in 28 days cycle, and
optionally wherein the anti-CD20 antibody is rituximab, ibritumomab tiuxetan, tositumomab, ofatumumab or obinutuzumab, preferably rituximab or obinutuzumab.

**Patentansprüche**

1. BTK-Inhibitor zur Verwendung in einem Verfahren zur Behandlung eines menschlichen Patienten mit einem diffusen großzelligen Nicht-GCB-B-Zell-Lymphom, wobei der Patient **gekennzeichnet ist durch** eine CD79B-Mutation und wobei der BTK-Inhibitor Verbindung 1 ist:

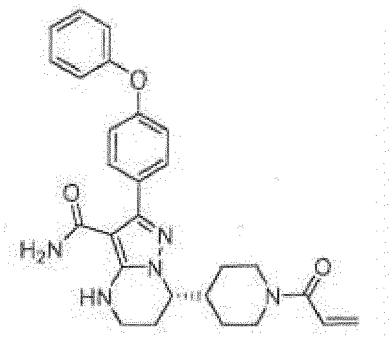

Verbindung 1.

2. BTK-Inhibitor zur Verwendung nach Anspruch 1, wobei Verbindung 1 in einer Menge von 320 mg einmal täglich verabreicht wird.

3. BTK-Inhibitor zur Verwendung nach Anspruch 1, wobei Verbindung 1 in einer Menge von 160 mg zweimal täglich verabreicht wird.

4. BTK-Inhibitor zur Verwendung in einem Verfahren zur Behandlung eines menschlichen Patienten mit einem diffusen großzelligen Nicht-GCB-B-Zell-Lymphom, wobei der BTK-Inhibitor in Kombination mit einem Anti-CD20-Antikörper verabreicht wird, wobei der Patient **gekennzeichnet ist durch** eine CD79B-Mutation und wobei der BTK-Inhibitor Verbindung 1 ist:

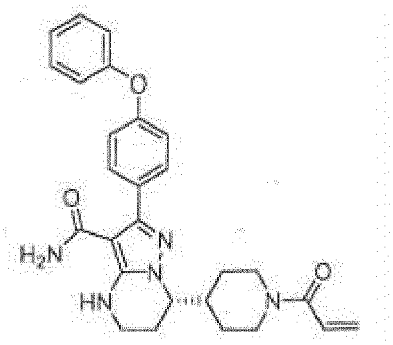

Verbindung 1.

5. BTK-Inhibitor zur Verwendung nach Anspruch 4, wobei Verbindung 1 in einer Menge von 320 mg einmal täglich verabreicht wird.

6. BTK-Inhibitor zur Verwendung nach Anspruch 4, wobei Verbindung 1 in einer Menge von 160 mg zweimal täglich verabreicht wird.

7. BTK-Inhibitor zur Verwendung nach Anspruch 2, 3, 5 oder 6, wobei Verbindung 1 in Zyklen von 28 Tagen verabreicht wird.

8. BTK-Inhibitor zur Verwendung nach Anspruch 4, wobei der Anti-CD20-Antikörper Rituximab, Ibritumomab-Tiuxetan, Tositumomab, Ofatumumab oder Obinutuzumab ist.

9. BTK-Inhibitor zur Verwendung nach Anspruch 8, wobei der Anti-CD20-Antikörper Rituximab ist.

10. BTK-Inhibitor zur Verwendung nach Anspruch 8, wobei der Anti-CD20-Antikörper Obinutuzumab ist.

11. Anti-CD20-Antikörper zur Verwendung in einem Verfahren zur Behandlung eines menschlichen Patienten mit einem diffusen großzelligen Nicht-GCB-B-Zell-Lymphom, wobei ein BTK-Inhibitor in Kombination mit dem Anti-CD20-Antikörper verabreicht wird, wobei der Patient **gekennzeichnet ist durch** eine CD79B-Mutation, wobei der BTK-Inhibitor Verbindung 1 von Anspruch 4 ist,

    wobei Verbindung 1 gegebenenfalls in einer Menge von 320 mg einmal täglich oder in einer Menge von 160 mg zweimal täglich verabreicht wird,
    wobei Verbindung 1 gegebenenfalls in einem Zyklus von 28 Tagen verabreicht wird und
    wobei der Anti-CD20-Antikörper gegebenenfalls Rituximab, Ibritumomab-Tiuxetan, Tositumomab, Ofatumumab oder Obinutuzumab, vorzugsweise Rituximab oder Obinutuzumab, ist.

**Revendications**

1. Inhibiteur de BTK à utiliser dans un procédé de traitement d'un patient humain atteint d'un lymphome diffus à grandes cellules B non GCB, dans lequel le patient est **caractérisé par** une mutation CD79B, et dans lequel l'inhibiteur de BTK est le composé 1 :

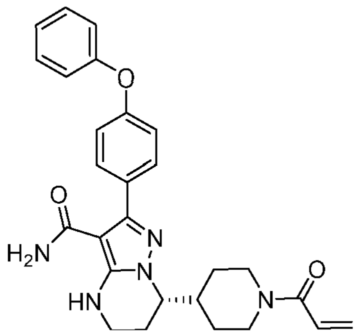

**Composé 1.**

2. Inhibiteur de BTK à utiliser selon la revendication 1, dans lequel le composé 1 est administré à 320 mg une fois par jour.

3. Inhibiteur de BTK à utiliser selon la revendication 1, dans lequel le composé 1 est administré à 160 mg deux fois par jour.

4. Inhibiteur de BTK à utiliser dans un procédé de traitement d'un patient humain atteint d'un lymphome diffus à grandes cellules B non GCB, dans lequel l'inhibiteur de BTK est administré en combinaison avec un anticorps anti-CD20, dans lequel le patient est **caractérisé par** une mutation CD79B, et dans lequel l'inhibiteur de BTK est le composé 1 :

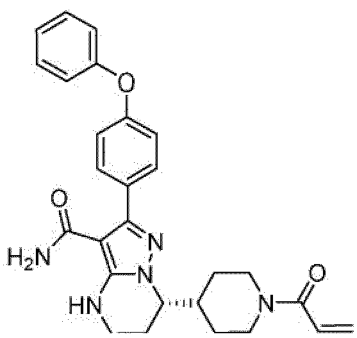

**Composé 1.**

**5.** Inhibiteur de BTK à utiliser selon la revendication 4, dans lequel le composé 1 est administré à 320 mg une fois par jour.

**6.** Inhibiteur de BTK à utiliser selon la revendication 1, dans lequel le composé 1 est administré à 160 mg deux fois par jour.

**7.** Inhibiteur de BTK à utiliser selon la revendication 2, 3, 5 ou 6, dans lequel le composé 1 est administré en cycles de 28 jours.

**8.** Inhibiteur de BTK à utiliser selon la revendication 4, dans lequel l'anticorps anti-CD20 est le rituximab, l'ibritumomab tiuxétan, le tositumomab, l'ofatumumab ou l'obinutuzumab.

**9.** Inhibiteur de BTK à utiliser selon la revendication 8, dans lequel l'anticorps anti-CD20 est le rituximab.

**10.** Inhibiteur de BTK à utiliser selon la revendication 8, dans lequel l'anticorps anti-CD20 est l'obinutuzumab.

**11.** Anticorps anti-CD20 à utiliser dans un procédé de traitement d'un patient humain atteint d'un lymphome diffus à grandes cellules B non GCB, dans lequel un inhibiteur de BTK est administré en combinaison avec l'anticorps anti-CD20, dans lequel le patient est **caractérisé par** une mutation CD79B, dans lequel l'inhibiteur de BTK est le composé 1 de la revendication 4,

facultativement dans lequel le composé 1 est administré à 320 mg une fois par jour ou à 160 mg deux fois par jour,
facultativement dans lequel le composé 1 est administré en cycle de 28 jours, et
facultativement dans lequel l'anticorps anti-CD20 est le rituximab, l'ibritumomab tiuxétan, le tositumomab, l'ofatumumab ou l'obinutuzumab, de préférence le rituximab ou l'obinutuzumab.

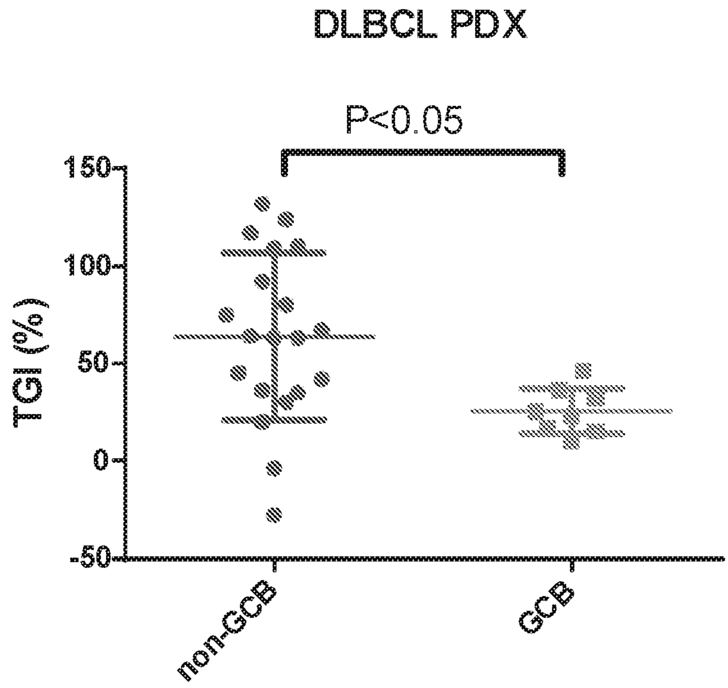


Figure 1

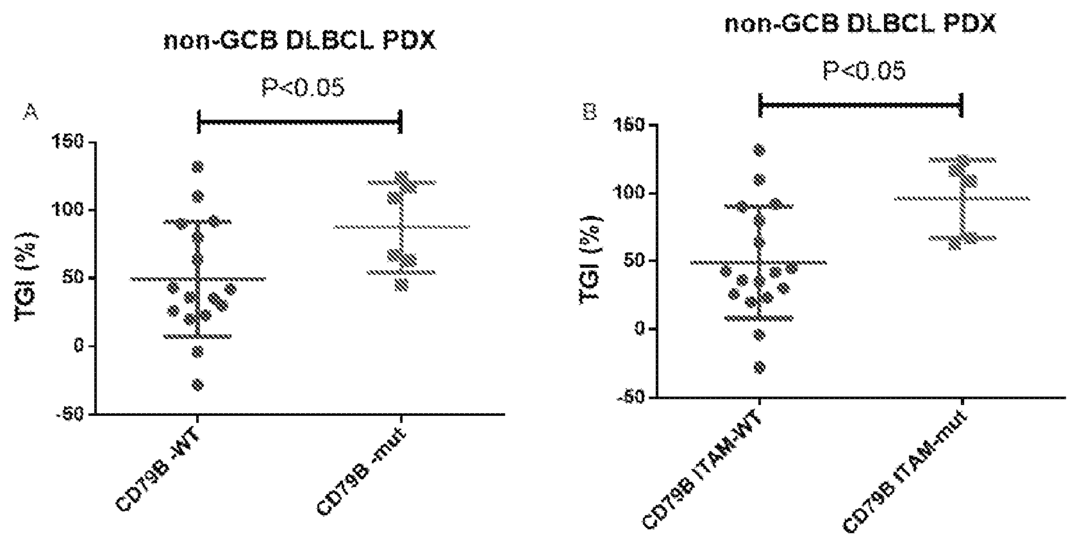


Figure 2

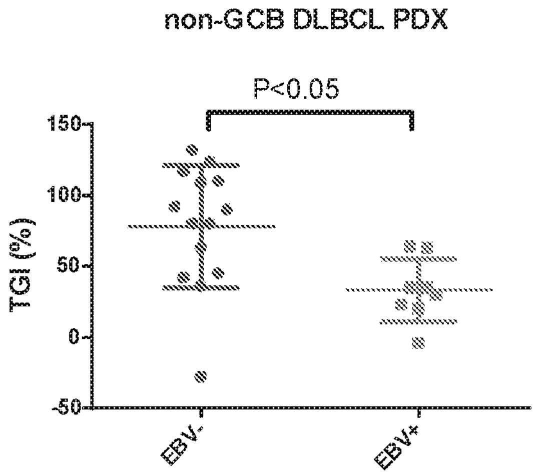


Figure 3

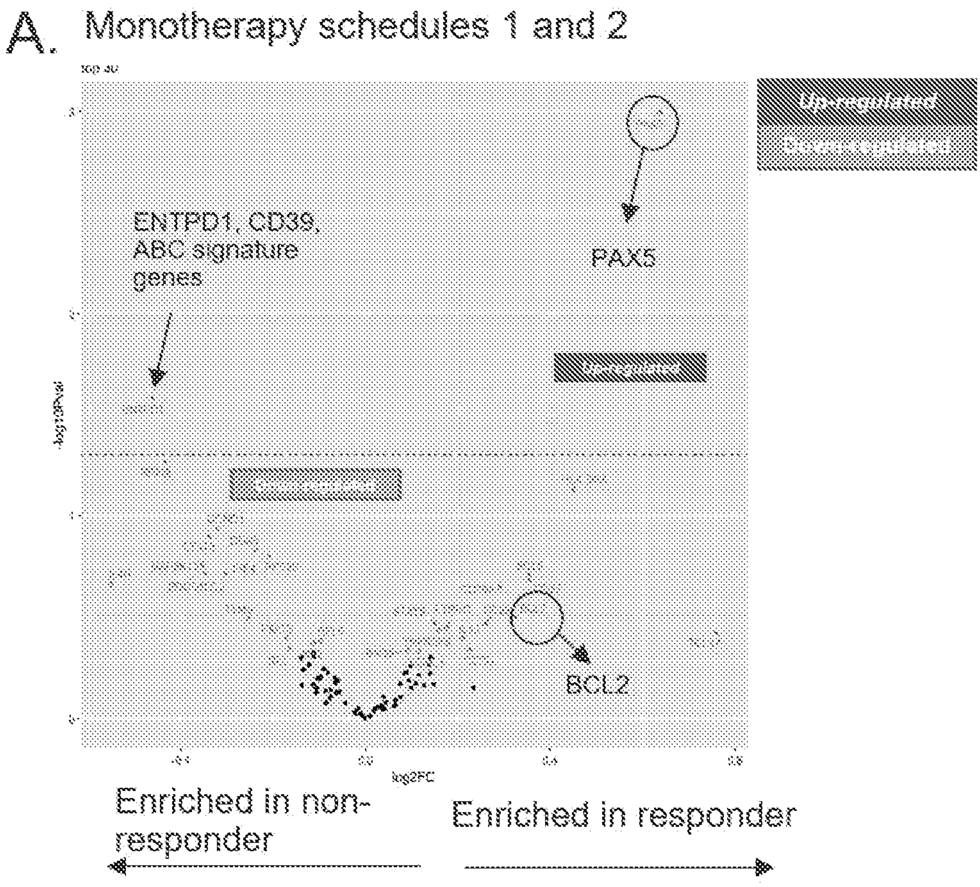


Figure 4(A)

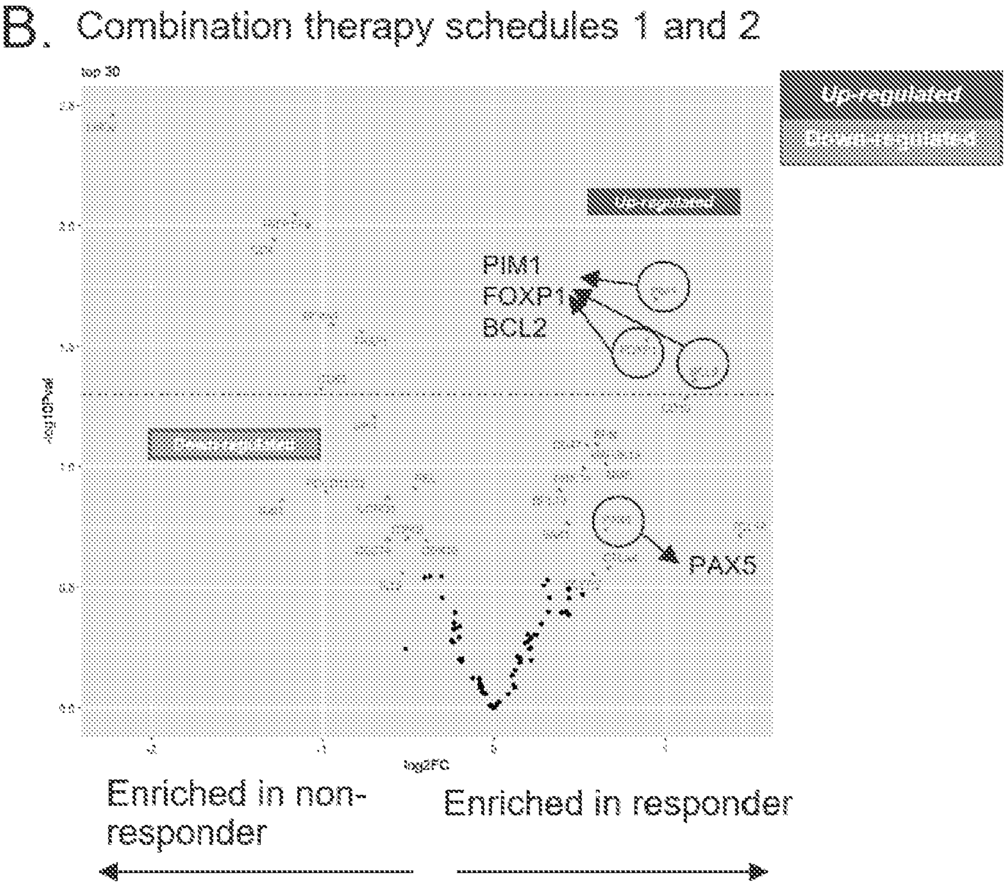
B. Combination therapy schedules 1 and 2
top 30
Up-regulated
PIM1
FOXP1
BCL2
PAX5
log2FC
-log10Pval
Enriched in non-responder
Enriched in responder

Figure 4(B)

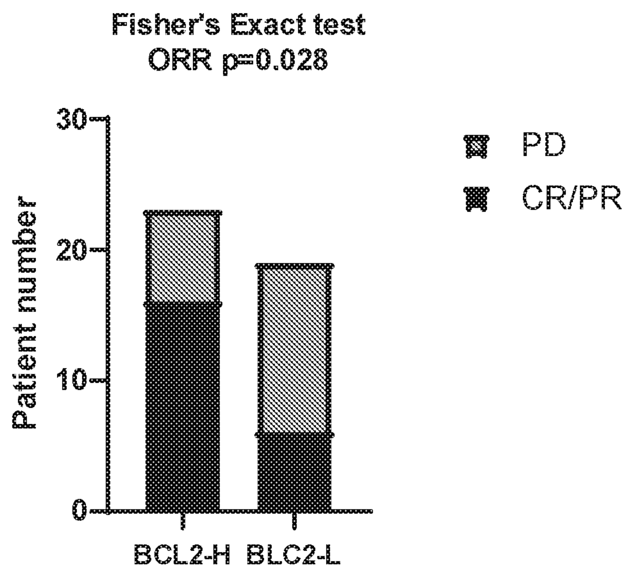


Figure 5 A

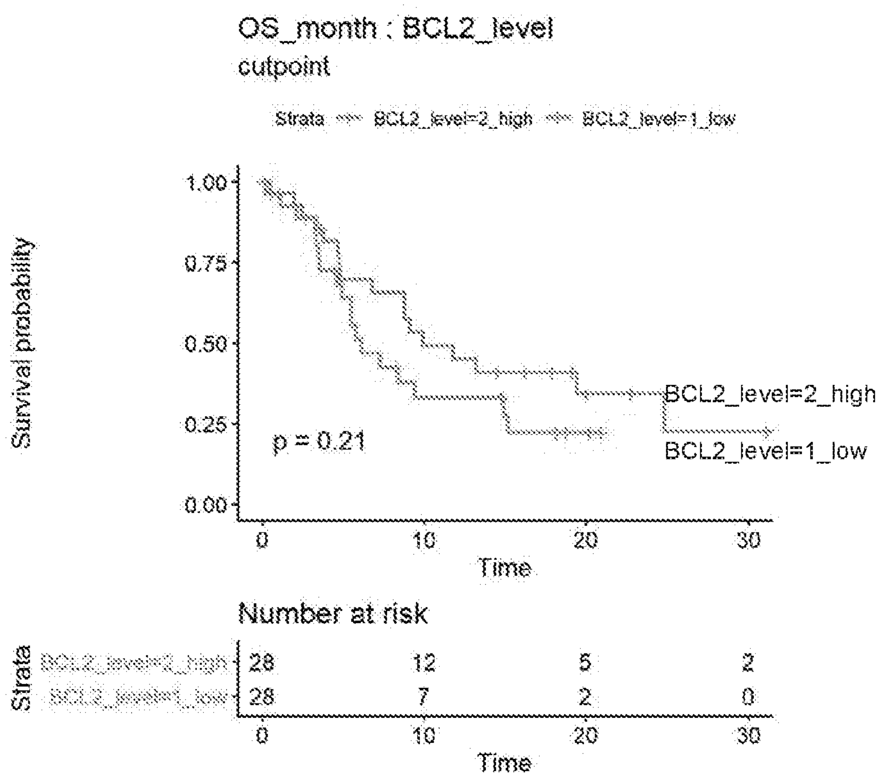


Figure 5 B

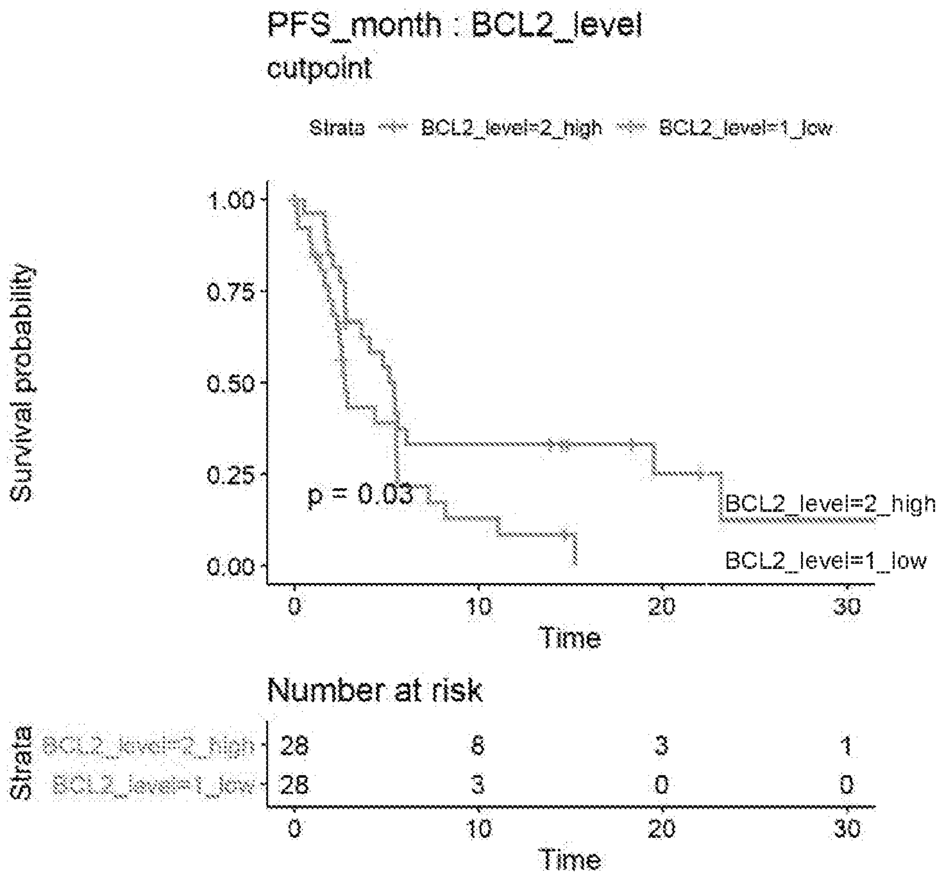


Figure 5 C

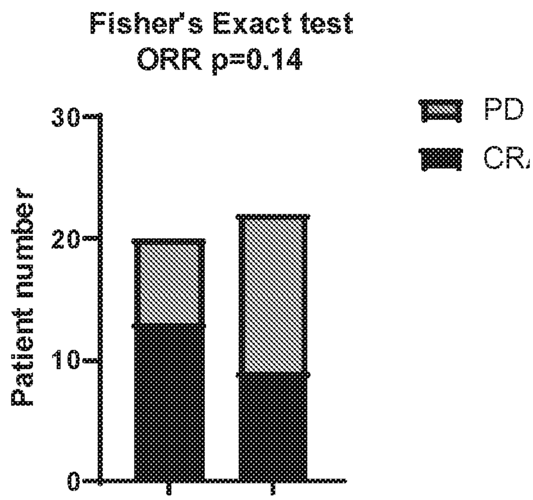


Figure 6 A

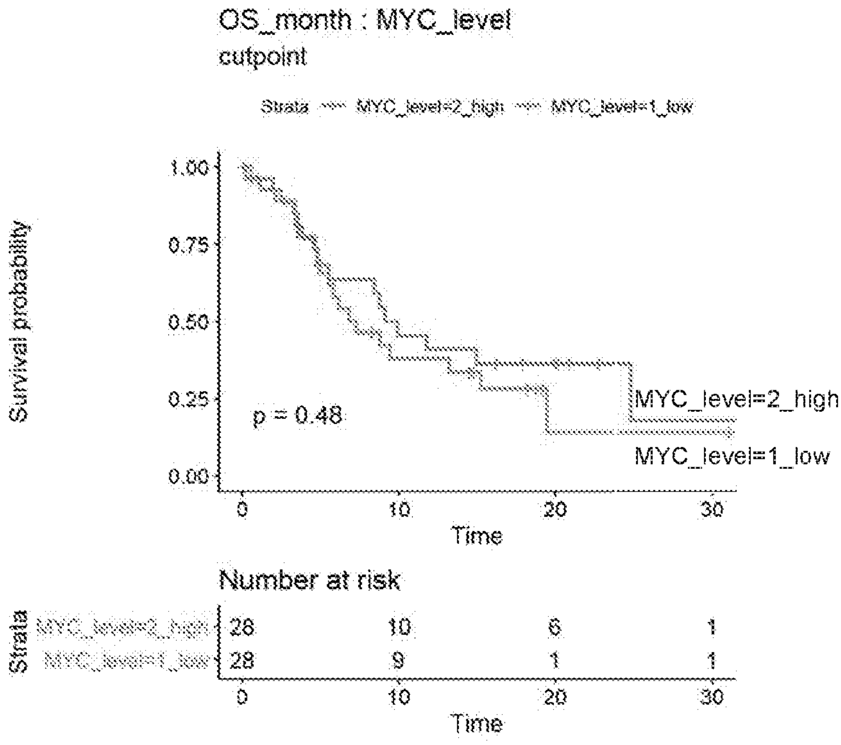


Figure 6 B

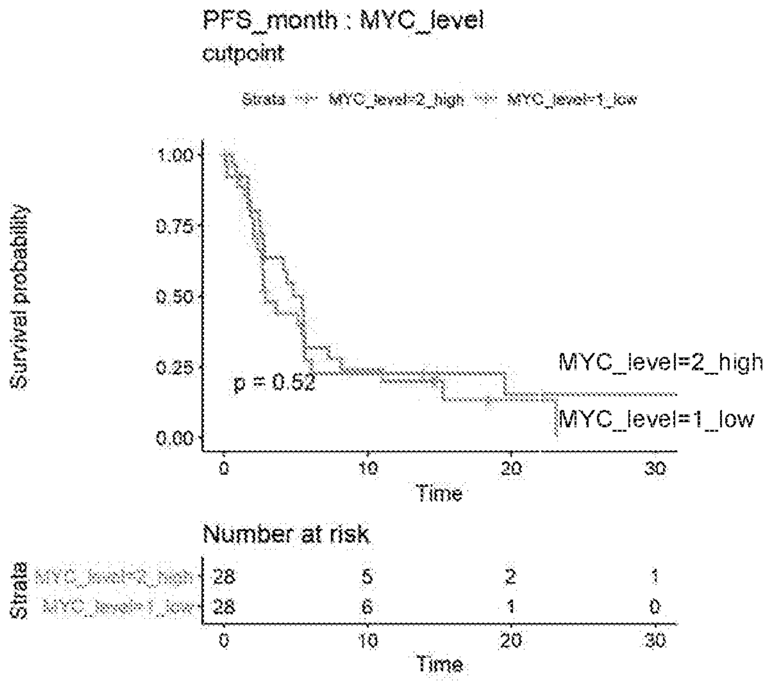


Figure 6 C

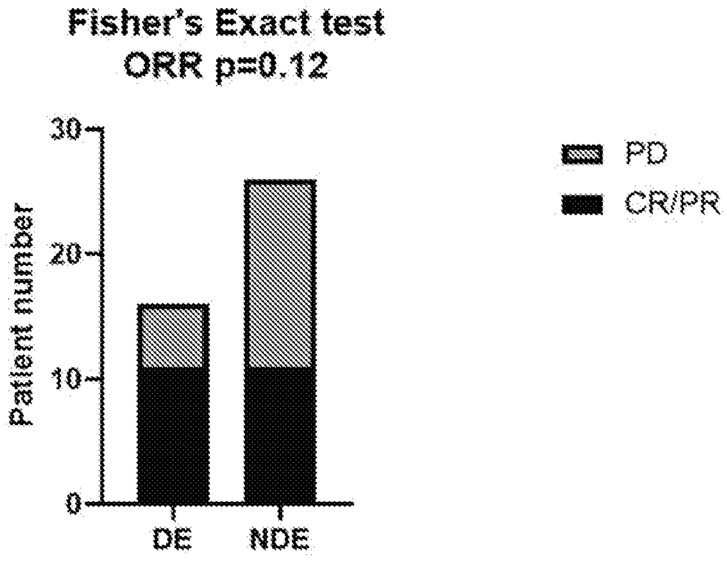


Figure 7 A

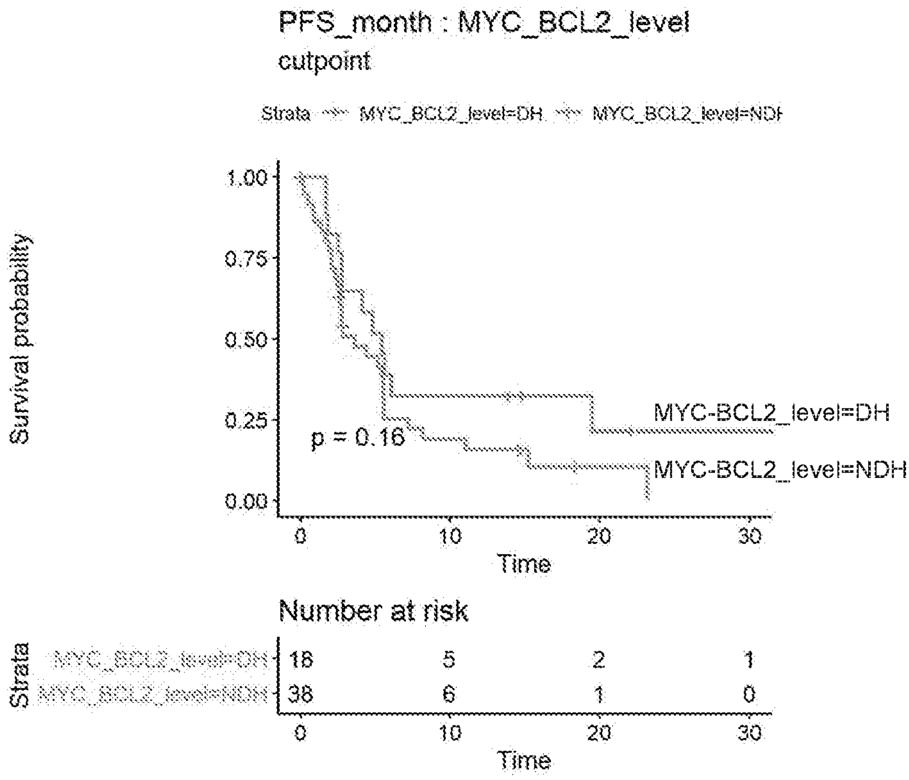


Figure 7 B

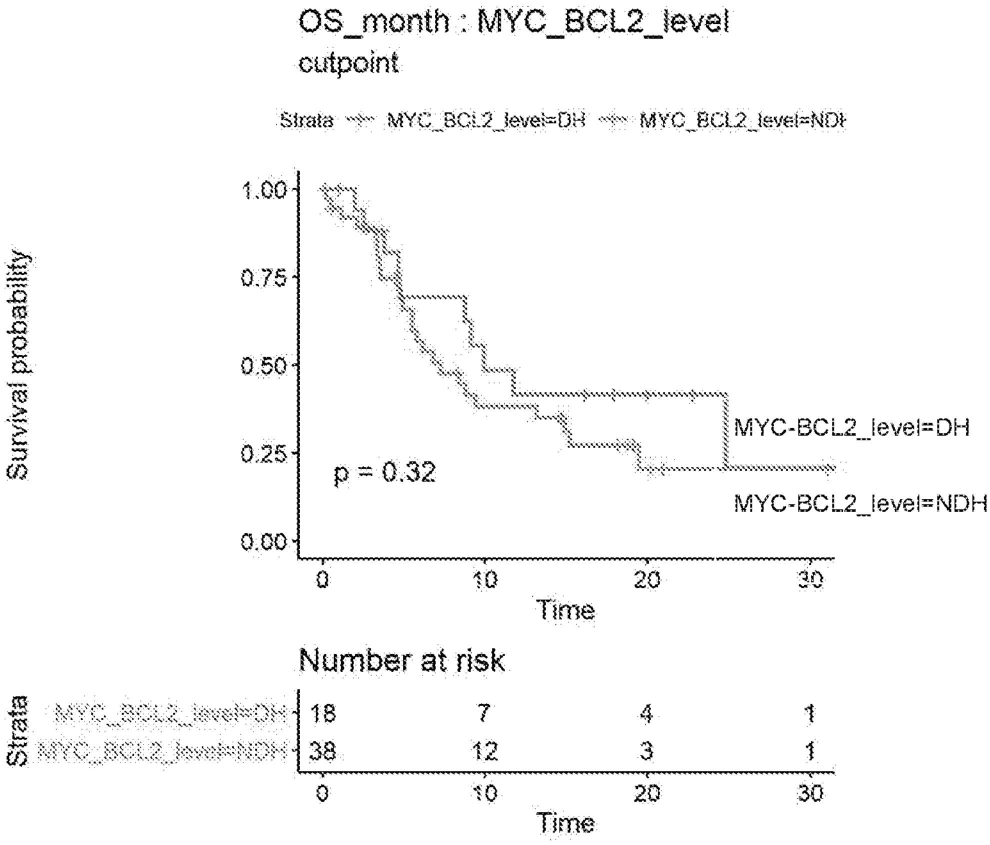
OS_month : MYC_BCL2_level
cutpoint
Strata MYC_BCL2_level=DH MYC_BCL2_level=NDH
Survival probability
1.00
0.75
0.50
0.25
0.00
p = 0.32
MYC-BCL2_level=DH
MYC-BCL2_level=NDH
0
10
20
30
Time
Number at risk
Strata
MYC_BCL2_level=DH 18 7 4 1
MYC_BCL2_level=NDH 38 12 3 1
0
10
20
30
Time

Figure 7 C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015127261 A
- WO 2014173289 A


### Non-patent literature cited in the description


- **ALIZADEH et al.** *Nature,* 2000, vol. 403 (6769), 503-11
- **ROSENWALD et al.** *N Engl J Med.,* 2002, vol. 346, 1937-1947
- **SWERDLOW et al.** *Blood,* 2016, vol. 127 (20), 2375-2390
- **AUKEMA et al.** *Blood,* 2011, vol. 117 (8), 2319-2331
- **HANS, C.P. et al.** *Blood,* 2004, vol. 103 (1), 275-282